Europäisches Patentamt

European Patent Office

Office européen des brevets

(11) Publication number: **0 055 885**
A2

(12) **EUROPEAN PATENT APPLICATION**

(21) Application number: **81304224.9**

(22) Date of filing: **15.09.81**

(51) Int. Cl.³: **A 61 K 37/26**

(30) Priority: **29.12.80 ZA 808097**

(43) Date of publication of application:
**14.07.82 Bulletin 82/28**

(84) Designated Contracting States:
**BE CH DE FR GB IT LI LU NL SE**

(71) Applicant: **ELI LILLY AND COMPANY**
**307, East McCarty Street**
**Indianapolis Indiana 46285(US)**

(72) Inventor: **Davies, Douglas Morgan**
**2635 Tournament Drive**
**Greenwood Indiana 46142(US)**

(74) Representative: **Crowther, Terence Roger et al,**
**Erl Wood Manor**
**Windlesham Surrey GU20 6PH(GB)**

(54) Novel insulin formulations.

(57) Disclosed are compositions suitable for pharmaceutical administration which comprise human insulin and accompanying adjuvants in a sterile aqueous diluent.

EP 0 055 885 A2

Croydon Printing Company Ltd.

## NOVEL INSULIN FORMULATIONS

The present invention relates to new insulin preparations. More particularly, this invention relates to insulin formulations containing human insulin.

For many years, insulin has been recognized as useful in treating diabetes mellitus. However, to date insulin pharmaceutical formulations have been prepared from insulin isolated from the bovine or porcine pancreases. Human insulin in practical quantities has not been available.

With the advent of recombinant DNA technology, practical quantities of human insulin have, for the first time, become available. It is to novel pharmaceutical compositions containing such insulin that this invention is directed.

Thus, this invention is directed to a pharmaceutical formulation comprising human insulin, a pharmaceutically acceptable diluent, and one or more adjuvants.

By the term "human insulin" is meant that insulin produced by the human pancreas under normal physiological conditions. The source of such human insulin is not significant to this invention. Thus, it may be isolated from the pancreas; it may be wholly synthetic; it may be semi-synthetic by conversion of animal insulin, particularly porcine insulin; it may be produced by recombinant DNA technology; or it may be derived from any other methodology.

The pharmaceutical compositions of this invention include a sterile, aqueous diluent, such as isotonic saline, and any of a number of adjuvants, such as buffers, preservatives, dispersing agents, agents that promote rapid onset of action or prolonged duration of action, and the like. Typical such adjuvants are, for example, phenol, m-cresol, glycerin, zinc oxide, sodium phosphate, sodium acetate, sodium chloride, methyl p-hydroxybenzoate, protamine sulfate, and the like.

In addition, an acid, such as hydrochloric acid, or a base, such as sodium hydroxide, can be used for pH adjustment. In general, the pH of the resulting composition ranges from about 6.8 to about 8.0.

As indicated hereinabove, the pharmaceutical compositions of this invention are useful in the treatment of diabetes mellitus. The dosage requirements for treating such condition depend, of course, upon its severity and, as such, will vary from case to case. Any particular dosage requirements thus will be a matter to be determined by the individual physician.

The specific insulin formulation to be administered to the patient will also be determined by the physician on a case by case basis. The physician may prescribe, for instance, a neutral regular insulin formulation or a protamine insulin formulation where a prolonged dose is desired. A modified formulation comprising a mixture of neutral regular insulin and protamine insulin may be prescribed for patients for whom a combination of the ready availability of the neutral regular insulin and the prolonged dose effect of the protamine insulin is advantageous. The two insulin components in the modified formulation can be mixed in any proportion, the ratio of protamine insulin to neutral regular insulin in the modified formulation being adjusted to achieve the desired physiological effect in the patient. A mixture of 70 parts by weight of protamine insulin and 30 parts by weight of neutral regular insulin has been used when animal insulin, particularly porcine insulin, is administered to the patient. A composition having an 70/30 ratio can be prepared, for example, by mixing 70 parts by weight of the composition of Example 6 with 30 parts by weight of the composition of Example 2.

Examples of particular pharmaceutical compositions of this invention are provided in the examples appearing hereinbelow.

Example 1 -- Neutral Regular Human Insulin Formulation: 40 Units (U) per Cubic Centimeter (cc.)

To prepare 10 cc. of the composition, mix

| | |
|---|---|
| Human Zinc Insulin (28 U/mg.) | 400 U |
| Phenol, distilled | 20 mg. |
| Glycerin | 160 mg. |

Water and either 10% hydrochloric acid or 10% sodium hydroxide sufficient to make a composition volume of 10 cc. and a final pH of 7.0-7.8.

X-5692                                    -4-

Example 2 -- Neutral Regular Human Insulin Formulation:
        100 U per cc.

    To prepare 10 cc. of the composition, mix

Human Zinc Insulin (28 U/mg.)          1000 U
Phenol, distilled                        20 mg.
Glycerin                                160 mg.

Water and either 10% hydrochloric
acid or 10% sodium hydroxide suf-
ficient to make a composition volume
of 10 cc. and a final pH of 7.0-7.8.

Example 3 -- Protamine, Zinc Human Insulin
        Formulation:  40 U per cc.

    To prepare 10 cc. of the composition, mix

Human Zinc Insulin (28 U/mg.)           400 U
Phenol, distilled                        25 mg.
Zinc Oxide                             0.78 mg.
Glycerin                                160 mg.
Protamine Sulfate                     4.0-6.0 mg.
Sodium Phosphate, crystals               38 mg.

Water and either 10% hydrochloric
acid or 10% sodium hydroxide suf-
ficient to make a composition
volume of 10 cc. and a final
pH of 7.1-7.4.

Example 4 -- Protamine, Zinc Human Insulin
         Formulation:  100 U per cc.

         To prepare 10 cc. of the composition, mix

| | |
|---|---|
| Human Zinc Insulin (28 U/mg.) | 1000 U |
| Phenol, distilled | 25 mg. |
| Zinc oxide | 2.0 mg. |
| Glycerin | 160 mg. |
| Protamine Sulfate | 10-15 mg. |
| Sodium Phosphate, crystals | 38 mg. |

Water and either 10% hydrochloric
acid or 10% sodium hydroxide suf-
ficient to make a composition
volume of 10 cc. and a final
pH of 7.1-7.4.

Example 5 -- Isophane Protamine, Zinc Human Insulin
         Formulation:  40 U per cc.

         To prepare 10 cc. of the composition, mix

| | |
|---|---|
| Human Zinc Insulin (28 U/mg.) | 400 U |
| m-Cresol, distilled | 16 mg. |
| Phenol, distilled | 6.5 mg. |
| Glycerin | 160 mg. |
| Protamine Sulfate | 1.2-2.4 mg. |
| Sodium Phosphate, crystals | 38 mg. |

Water and either 10% hydrochloric
acid or 10% sodium hydroxide suf-
ficient to make a composition volume
of 10 cc. and a final pH of 7.1-7.4.

X-5692                              -6-

Example 6 -- Isophane Protamine, Zinc Human Insulin
            Formulation:  100 U per cc.

To prepare 10 cc. of the composition, mix

| | |
|---|---|
| Human Zinc Insulin (28 U/mg.) | 1000 U |
| m-Cresol, distilled | 16 mg. |
| Phenol, distilled | 6.5 mg. |
| Glycerin | 160 mg. |
| Protamine Sulfate | 3.0-6.0 mg. |
| Sodium Phosphate, Crystals | 38 mg. |

Water and either 10% hydrochloric
acid or 10% sodium hydroxide suf-
ficient to make a composition
volume of 10 cc. and a final pH
of 7.1-7.4.

Example 7 -- Human Insulin Zinc Suspension Formulation:
            40 U per cc.

To prepare 10 cc. of the composition, mix

| | |
|---|---|
| Human Zinc Insulin (28 U/mg.) | 400 U |
| Sodium Acetate, Anhydrous | 16 mg. |
| Sodium Chloride, Granular | 70 mg. |
| Methyl p-Hydroxybenzoate | 10 mg. |
| Zinc Oxide | 0.63 mg. |

Water and either 10% hydrochloric
acid or 10% sodium hydroxide suf-
ficient to make a composition
volume of 10 cc. and a final
pH of 7.2-7.5.

Example 8 -- Human Insulin Zinc Suspension Formulation:
          100 U per cc.

          To prepare 10 cc. of the composition, mix

| | |
|---|---|
| Human Zinc Insulin (28 U/mg.) | 1000 U |
| Sodium Acetate, Anhydrous | 16 mg. |
| Sodium chloride, Granular | 70 mg. |
| Methyl p-Hydroxybenzoate | 10 mg. |
| Zinc Oxide | 1.6 mg. |

Water and either 10% hydrochloric acid or 10% sodium hydroxide sufficient to make a composition volume of 10 cc. and a final pH of 7.2-7.5.

X-5692                          -8-

## CLAIMS

1. A composition suitable for pharmaceutical administration which comprises human insulin and accompanying adjuvants in a sterile aqueous diluent.

2. The composition of claim 1 which comprises human zinc insulin.

3. The composition of claim 1 or 2 in which the adjuvants comprise protamine sulfate.

4. The composition of claim 3 which is prepared by mixing a neutral regular human insulin formulation and a protamine human insulin.

5. The composition of claim 4 which is prepared by mixing 30 parts by weight of neutral regular human insulin and 70 parts by weight of protamine human insulin.